(19) Europäisches Patentamt
European Patent Office
Office européen des brevets

(11) **EP 1 314 417 A1**

(12) **EUROPEAN PATENT APPLICATION**
published in accordance with Art. 158(3) EPC

(43) Date of publication:
**28.05.2003 Bulletin 2003/22**

(21) Application number: **01963408.8**

(22) Date of filing: **31.08.2001**

(51) Int Cl.[7]: **A61K 7/06**, A61P 17/14

(86) International application number:
**PCT/JP01/07538**

(87) International publication number:
**WO 02/017863 (07.03.2002 Gazette 2002/10)**

(84) Designated Contracting States:
**AT BE CH CY DE DK ES FI FR GB GR IE IT LI LU MC NL PT SE TR**

(30) Priority: **31.08.2000 JP 2000262996**

(71) Applicant: **Shiseido Co., Ltd.**
**Tokyo 104-8010 (JP)**

(72) Inventors:
- **TAKAHASHI, Tadahito**
  **Tsuzuki Yokohama Kanagawa 224-8558 (JP)**
- **NAKAZAWA, Yosuke**
  **Tsuzuki Yokohama Kanagawa 224-8558 (JP)**
- **ARAI, Takayuki**
  **Tsuzuki Yokohama Kanagawa 224-8558 (JP)**
- **IWABUCHI, Tokuro**
  **Shiseido Res. Ctr. Kanazawahakkei**
  **Yokohama-shi Kanagawa 236-8643 (JP)**
- **MAGARA, Tsunao**
  **Tsuzuki Yokohama Kanagawa 224-8558 (JP)**
- **FUKUNISHI, Hirotada**
  **Tsuzuki Yokohama Kanagawa 224-8558 (JP)**
- **KOBAYASHI, Koji**
  **Tsuzuki Yokohama Kanagawa 224-8558 (JP)**
- **TAJIMA, Masahiro**
  **Tsuzuki Yokohama Kanagawa 224-8558 (JP)**
- **OHNO, Shigeo**
  **Tokyo 152-0023 (JP)**
- **HANDA, Hiroshi**
  **Tokyo 156-0045 (JP)**

(74) Representative: **Gudat, Axel**
**Patentanwälte**
**Lippert, Stachow, Schmidt & Partner**
**Frankenforster Strasse 135-137**
**51427 Bergisch Gladbach (DE)**

(54) **HAIR GROWERS**

(57) The present invention provides a hair growth promoting composition having an excellent hair growth promoting effect. The hair promoting composition comprises, as an effective ingredient, a composition expressed by following Formula (I):

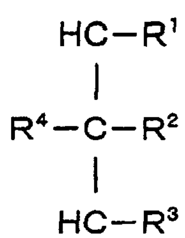

(I)

wherein one of $R^1$ to $R^4$ is selected from a group of $C_{14-22}$ alkyl, $C_{14-22}$ alkoxy and $C_{14-22}$ acyloxy groups, and the others are selected from a group of H, OH, $C_{1-3}$ alkyl and $C_{1-3}$ alkoxy groups; and when $R^1$ is $C_{14-22}$ alkoxy group and one of $R^2$ and $R^4$ is $C_{1-3}$ alkoxy group, $R^3$ is H, $C_{1-3}$ alkyl or $C_{1-3}$ alkoxy group; and when $R^1$ is $C_{14-22}$ acyloxy group, at least one of $R^2$ to $R^4$ is $C_{1-3}$ alkoxy group.

EP 1 314 417 A1

## Description

This application claims the priority of Japanese Patent Application No. 2000-262996 filed on August 31, 2000, which is incorporated herein by reference.

## FIELD OF THE INVENTION

The present invention relates to a hair growth promoting composition and, in particular, to an active ingredient thereof.

## BACKGROUND OF THE INVENTION

Conventionally, scalp abnormality due to activation of androgen in an organ such as hair root or sebaceous gland, lowering of blood stream toward hair follicle, excess secretion of sebum, formation of peroxide and the like has been considered as a cause of baldness or hair loss. Accordingly, a compound that can remove or reduce the above-mentioned problems has been generally included into a hair growth promoting composition (it is also called a hair growth composition or a hair regrowth promoting composition). For example, a vitamin such as Vitamin B or Vitamin E, an amino acid such as serine or methionine, a vasodilator such as swertia herb extract or acetylcholine derivatives, an antiinflammatory agent such as Lithospermum root extract or hinokitiol, a female hormone such as estradiol, and a cutaneous hyperfunctioning agent such as cepharanthine have been compounded and used for preventing and caring hair loss.

However, although the compounds described above have been compounded to the conventional hair growth promoting compositions to conduct various trials, they do not always exhibit sufficient hair growth promoting effect such as hair loss preventing effect or hair regrowthing effect, because hair loss has diverse causes and hair regrowth mechanism is very complicated.

## SUMMARY OF THE INVENTION

The present invention has been performed in view of the foregoing problem in the prior art and the object is to provide a hair growth promoting composition which can exhibit excellent hair growth promoting effect.

As a result of diligent studies of the inventors for attaining the above mentioned objects, it has been found that certain compounds have excellent hair growth promoting effect, thereby accomplishing the present invention.

Namely, a hair growth promoting composition in accordance with the present invention is characterized by comprising, as an effective ingredient, a compound expressed by the following Formula (I):

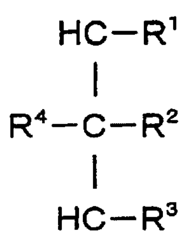

(I)

wherein one of $R^1$ to $R^4$ is selected from a group of $C_{14-22}$ alkyl, $C_{14-22}$ alkoxy and $C_{14-22}$ acyloxy groups, and the others are selected from a group of H, OH, $C_{1-3}$ alkyl and $C_{1-3}$ alkoxy groups;

when one of $R^1$ and $R^3$ is $C_{14-22}$ alkoxy group and one of $R^2$ and $R^4$ is $C_{1-3}$alkoxy group, the other of $R^1$ and $R^3$ is H, $C_{1-3}$alkyl or $C_{1-3}$alkoxy group; and

when one of $R^1$ and $R^3$ is $C_{14-22}$ acyloxy group, the compound of Formula (I) has at least one $C_{1-3}$alkoxy group therein.

In the present invention, $R^1$ is preferably $C_{14-22}$ alkyl group. It is more preferable that $R^1$ is heptadecyl group and $R^2$ is methoxy group and $R^3$ is OH, or that $R^1$ is heptadecyl group and $R^2$ and $R^3$ are OH.

In the present invention, $R^1$ is preferably $C_{14-22}$ acyloxy group. It is more preferable that $R^1$ is octadecanoyloxy

group and $R^2$ is methoxy group and $R^3$ is OH.

In the present invention, $R^2$ is preferably a $C_{14-22}$ alkoxy group. It is more preferable that $R^2$ is hexadecyloxy group and $R^1$ is methoxy group and $R^3$ is OH, or that $R^2$ is hexadecyloxy group and $R^1$ and $R^3$ are OH.

In the present invention, $R^1$ is preferably $C_{14-22}$ alkoxy group. It is more preferable that $R^1$ is hexadecyloxy group and $R^2$ and $R^3$ are methoxy groups, or that $R^1$ is hexadecyloxy group and $R^2$ is OH and $R^3$ is methoxy group, or that $R^1$ is hexadecyloxy group and $R^2$ is H and $R^3$ is OH.

In any of the above-mentioned hair growth promoting compositions, $R^4$ is preferably H.

In the present invention, it is preferable that $R^1$ is octadecyloxy group, $R^2$ and $R^4$ are methyl groups, and $R^3$ is OH.

A method for promoting hair growth in accordance with the present invention is characterized by applying an effective amount of said composition on the skin of mammals and, in particular, on human scalp.

## BEST MODE FOR CARRYING OUT THE INVENTION

In the present invention, the $C_{14-22}$ alkyl group may be straight, branched or cyclic group. It is preferably straight or branched $C_{16-18}$ alkyl group, more preferably heptadecyl group.

The $C_{14-22}$ alkoxy group means a hydroxy group which hydrogen atom is substituted by $C_{14-22}$ alkyl group mentioned above. It is preferably a strait or branched $C_{16-18}$ alkoxy group, more preferably hexadecyloxy or octadecyloxy group.

The $C_{14-22}$ acyloxy group means a hydroxy group which hydrogen atom is substituted by a carbonyl group having $C_{13-21}$ alkyl group. In other wards, it means $C_{14-22}$ alkanoyloxy group. It is preferably $C_{16-18}$ acyloxy group, more preferably octadecanoyloxy group.

The $C_{1-3}$ alkyl group may be straight, branched or cyclic group. It is preferably methyl group.

The $C_{1-3}$ alkoxy group means a hydroxy group which hydrogen atom is substituted by $C_{1-3}$ alkyl group mentioned above. It is preferably methoxy group.

The Compound(I) of the present invention may have a asymmetric center. The present invention can use optical isomers based on such asymmetric carbon and the mixture thereof. Also, when there are the other isomers, the present invention can include them.

Preferable examples of Compound (I) are as follows:

Compound 1 : 2-Hexadecyloxy-3-methoxy-1-propanol
Compound 2 : 1-Hexadecyloxy-3-methoxy-2-propanol
Compound 3 : 3-Hexadecyloxy-1-propanol
Compound 4 : 1-(2,3-Dimethoxypropoxy)hexadecane
Compound 5 : 2-Methoxy-l-eicosanol
Compound 6 : 3-Hydroxy-2-methoxypropyl stearate
Compound 7 : 2-Hexadecyloxy-1,3-propanediol
Compound 8 : 2,2-Dimethyl-3-octadecyloxy-1-propanol
Compound 9 : 1,2-eicosanediol

Although the above-described Compounds 1 to 9 have been known, their hair growth promoting effects and hair anagen stage prolonging effects have not been known and first found by the inventors.

Compound (I) may be manufactured by a chemical synthesis or may be obtained by extraction from a microorganism, a animal cell and plant cell in which it is produced. In the following, as a representative example, the chemical synthetic methods of Compounds 1 to 9 mentioned above will be explained. Compound 10 used in chemical synthesis of Compounds 1 to 9 was manufactured by Fluka. Compounds 14, 17, 21 and 24 were manufactured by TOKYO KASEI KOGYO CO., Ltd.. As for other regents, those on sale were used. The proton magnetic resonance spectrum ($^1$H-NMR) was measured using a JOEL EX-400 (400MHz) with a tetramethylsilane (TMS) as an internal standard.

Synthetic example 1 Synthesis of Compound 1

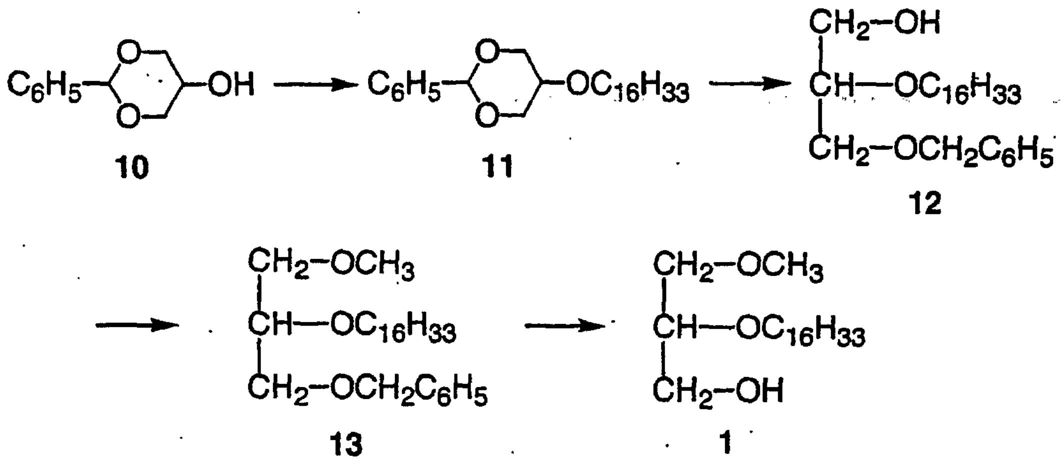

10 11 12 13 1

(1) Synthesis of Compound 11

To a solution of Compound 10(1.80 g, 10.00 mmol) in N,N-dimethylformamide (18 ml) was added sodium hydride(60% content in oil)(0.44 g, 11.00 mmol) in argon atmosphere and stirred for an hour while being cooled with ice. Then, the mixture with 1-bromohexadecane(3.66 g, 12.00 mmol) added thereto was stirred for 17 hours at room temperature. The reaction mixture was diluted with water, extracted with ethyl acetate, washed with saturated brine, dried over sodium sulfate and concentrated. The residue was purified by silica gel column chromatography(silica gel 92 g, hexane - hexane:ethyl acetate=10:1), to give Compound 11a(1.05 g, 26%) and Compound 11b(0.50 g, 12%) as white solids. Compound 11a and Compound 11b are stereoisomers each other.

Compound11a, $^1$H-NMR ($CDCl_3$) δ : 0.88 (3H, t, J=6.8 Hz), 1.26 (26H, m), 1.50 - 1.65 (2H, m), 3.38 (2H, t, J=6.8 Hz), 3.55 - 3.75 (3H, m), 4.38 (2H, dd, J=4.2,10.5 Hz), 5.39 (1H, s), 7.30 - 7.40 (3H, m), 7.40 - 7.50 (2H, m).

Compound 11b, $^1$H-NMR ($CDCl_3$) δ: 0.88 (3H, t, J=6.8 Hz), 1.26 (26H, m), 1.65 (2H, m), 3.25 (1H. s), 3.54 (2H, t, J=6.8 Hz), 4.04 (2H, dd, J=1.0,12.2 Hz), 4.33 (2H, d, J=12.2 Hz), 5.55 (1H, s), 7.30 - 7.40 (3H, m), 7.51 (2H, dd, J=1.7,7.6 Hz).

(2) Synthesis of Compound 12

To a solution of Compound 11 (mixture of Compound 11a and Compound 11b)(2.85 g, 7.04 mmol) in toluene (10 ml) was added 1.0M diisobutylaluminum hydride (DIBAL-H) in toluene(17.7 ml, 17.70 mmol) in argon atmosphere while being cooled with ice and stirred for 15 hours at room temperature. After methanol was dropwise added to the reaction mixture while being cooled with ice, 10% sodium hydroxide and water were added thereto. The mixture was extracted with ethyl acetate, washed with saturated brine, dried over sodium sulfate and concentrated. The residue was purified by silica gel column chromatography(silica gel 80 g, hexane:ethyl acetate=20:1-10:1-5:1), to give Compound 12(2.75 g, 96%) as a colorless oil.

$^1$H-NMR ($CDCl_3$) δ : 0.88 (3H, t, J=6.8 Hz), 1.26 (26H, m), 1.57 (2H, m), 2.10 (1H, t, J=6.4 Hz), 3.45 - 3.70 (6H, m), 3.70 - 3.80 (1H, m), 4.54 (2H, s), 7.20 - 7.40 (5H, m).

(3) Synthesis of Compound 13

To a solution of Compound 12(2.73 g, 6.71 mmol) in N,N- dimethylformamide(28 ml) was added sodium hydride(60% content in oil)(0.30 g, 7.38 mmol) and stirred for an hour while being cooled with ice. Then, methyl iodide (1.14 g, 8.06 mmol) was added to the mixture and stirred for 14 hours at room temperature. The reaction mixture was diluted with water, extracted with ethyl acetate, washed with saturated brine, dried over sodium sulfate and concentrated. The residue was purified by silica gel column chromatography(silica gel 90 g, hexane - hexane:ethyl acetate=10:1), to give Compound 13(2.34 g, 83%) as a colorless oil.

$^1$H-NMR ($CDCl_3$) δ : 0.88 (3H, t, J=6.8 Hz), 1.26 (26H, m), 1.57 (2H, m), 3.36 (3H, s), 3.40 - 3.65 (7H,m), 4.55 (2H, s), 7.20 - 7.40 (5H, m).

(4) Synthesis of Compound 1

To a solution of Compound 13(2.30 g, 5.47 mmol) in tetrahydrofuran (23 ml) was added 10% palladium-carbon (50% water included)(0.46 g) and stirred for 20 hours in hydrogen atmosphere at room temperature. The insoluble matters were filtrated out and the reaction mixture was concentrated. The obtained residue was purified by silica gel column chromatography(silica gel 60 g, hexane:ethyl acetate=10:1-5:1), to give Compound 1(1.55 g, 86%) as a white wax.

$^1$H-NMR ($CDCl_3$) $\delta$ : 0.88 (3H, t, J=6.8 Hz), 1.26 (26H, m), 1.58 (2H, m), 2.15 (1H, t, J=6.1 Hz), 3.37 (3H, s), 3.40 - 3.55 (4H, m), 3.57 - 3.66 (2H, m), 3.68 - 3.77 (1H, m).

Synthetic example 2 Synthesis of Compound 2

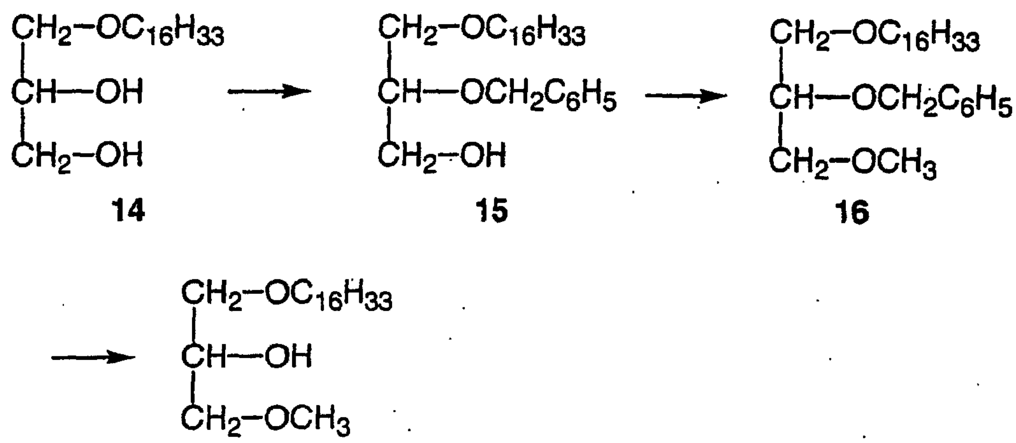

14 15 16

2

(1) Synthesis of Compound 15

To a suspension of Compound 14(1.00 g, 3.16 mmol) in toluene(10 ml) were added pyridine(0.28 ml, 3.48 mmol) and trityl chloride(0.88 g, 3.16 mmol). After being stirred for 21 hours at 115°C, the reaction mixture was diluted with ethyl acetate, washed with 1N hydrochloric acid, water and saturated brine successively, dried over sodium sulfate and concentrated. The residue was purified by silica gel column chromatography(silica gel 20 g, chloroform) and concentrated. To a solution of the obtained residue(1.36 g) in N,N-dimethylformamide(10 ml) was added sodium hydride (60% content in oil)(0.11 g, 2.68 mmol), and stirred for an hour while being cooled with ice. The reaction mixture, with benzyl bromide (0.35 ml, 2.92 mmol) added thereto, was stirred for 16 hours at room temperature. The reaction mixture was diluted with ethyl acetate, washed with water and saturated brine successively, dried over sodium sulfate and concentrated. The residue was purified by silica gel column chromatography(silica gel 40 g, hexane - hexane:ethyl acetate=50:1) and concentrated. Then, to the residue (1.58 g) were added triethyl borate(3 ml) and boric acid(1.50 g, 24.26 mmol) and stirred for 3 hours at 120°C. The reaction mixture was diluted with water, extracted with chloroform, dried over sodium sulfate and concentrated. The residue was purified by silica gel column chromatography(silica gel 40 g, hexane:ethyl acetate=50:1-4:1), to give Compound 15(0.64 g, 50%) as a colorless oil.

$^1$H-NMR ($CDCl_3$) $\delta$ : 0.88 (3H, t, J=6.8 Hz), 1.26 (26H, m), 1.56 (2H, m), 2.16 (1H, t, J=5.4 Hz), 3.44 (2H, dt, J=1.5, 6.7 Hz), 3.54 (1H, dd, J=5.4, 9.8 Hz), 3.59 (1H, dd, J=4.7, 9.8 Hz), 3.67 (2H, m), 3.74 (1H, m), 4.62 (1H, d, J=11.7 Hz), 4.71 (1H, d, J=11.7 Hz), 7.20 - 7.45 (5H, m).

(2) Synthesis of Compound 16

To a solution of Compound 15(0.64 g, 1.57 mmol) in N,N-dimethylformamide(4 ml) was added sodium hydride (60% content in oil)(0.08 g, 1.89 mmol) and stirred for an hour while being cooled with ice. Methyl iodide(0.12 ml, 1.89 mmol) was added to the mixture and stirred for an hour at room temperature. The reaction mixture was diluted with water and then concentrated. The residue was diluted with ethyl acetate, washed with water, dried over sodium sulfate and concentrated. The residue was purified by silica gel column chromatography(silica gel 20 g, hexane - hexane:ethyl

acetate=5:1), to give Compound 16(0.58 g, 88%) as a colorless oil.

$^1H$-NMR ($CDCl_3$) $\delta$ : 0.88 (3H, t, J=6.8 Hz), 1.26 (26H, m), 1.56 (2H, m), 3.36 (3H, s), 3.43 (2H, t, J=6.6 Hz), 3.46 - 3.57 (4H, m), 3.72 (1H, m), 4.70 (2H, s), 7.20 - 7.45 (5H, m).

(3) Synthesis of Compound 2

To a solution of Compound 16(0.58 g, 1.38 mmol) in ethanol(5 ml) was added 10% palladium-carbon(50% water content)(0.12 g) and stirred for 15 hours in hydrogen atmosphere at room temperature. After the insoluble maters were filtrated out, the filtrate was concentrated. The obtained residue was purified by silica gel column chromatography (silica gel 10 g, hexane:ethyl acetate=4:1), to give Compound 2(0.45 g, 99%) as a white wax.

$^1H$-NMR ($CDCl_3$) $\delta$ : 0.88 (3H, t, J=6.8 Hz), 1.26 (26H, m), 1.57 (2H, m), 2.51 (1H, d, J=3.4 Hz), 3.39 (3H, s), 3.40 - 3.55 (6H, m), 3.95 (1H, m).

Synthetic example 3 Synthesis of Compound 3

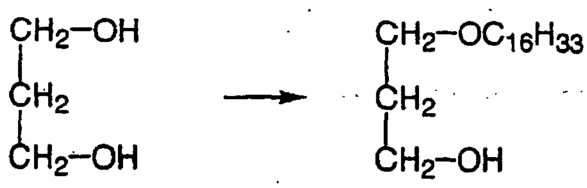

17 3

To a solution of Compound 17(0.76 g, 10.00 mmol) in N,N-dimethylformamide(10 ml) was added sodium hydride(60% content in oil)(0.88 g, 22.00 mmol) and stirred for an hour while being cooled with ice. 1-Iodohexadecane (3.14 ml, 10.00 mmol) was added to the mixture and stirred for 14 hours at room temperature. The reaction mixture was diluted with water and then concentrated. The obtained residue was diluted with ethyl acetate, washed with water, dried over sodium sulfate, and concentrated. The residue was purified by silica gel column chromatography(silica gel 50 g, hexane:ethyl acetate=5:1), to give Compound 3(1.13 g, 38%) as a white crystal.

$^1H$-NMR ($CDCl_3$) $\delta$ : 0.88 (3H, t, J=6.8 Hz), 1.26 (26H, m), 1.56 (2H, m), 1.82 (2H, m), 2.71 (1H, br s), 3.42 (2H, t, J=6.8 Hz), 3.60 (2H, t, J=5.9 Hz), 3.76 (2H, br t, J=5.4 Hz).

Synthetic example 4 Synthesis of Compound 4

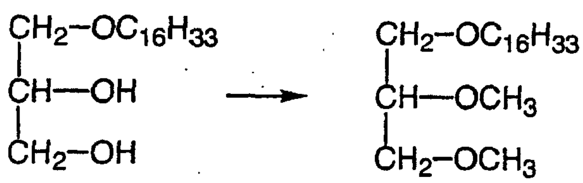

14 4

To a solution of Compound 14(1.00 g, 3.16 mmol) in tetrahydrofuran(10 ml) was added sodium hydride(60% content in oil)(0.28 g, 6.95 mmol) and stirred for 1 hour while being cooled with ice. Methyl iodide(0.20 ml, 3.16 mmol) was added to the mixture and stirred for 14 hours at room temperature. The reaction mixture was diluted with water, extracted with ethyl acetate, dried over sodium sulfate, and then concentrated. The residue was purified by silica gel column chromatography(silica gel 40 g, hexane - hexane:ethyl acetate=5:1) , to give Compound 4(0.79 g, 73%) as a colorless oil.

$^1$H-NMR ($CDCl_3$) δ : 0.88 (3H, t, J=6.8 Hz), 1.26 (26H, m), 1.57 (2H, m), 3.37 (3H, s), 3.40 - 3.55 (7H, m), 3.47 (3H, s).

Synthetic example 5 Synthesis of Compound 5

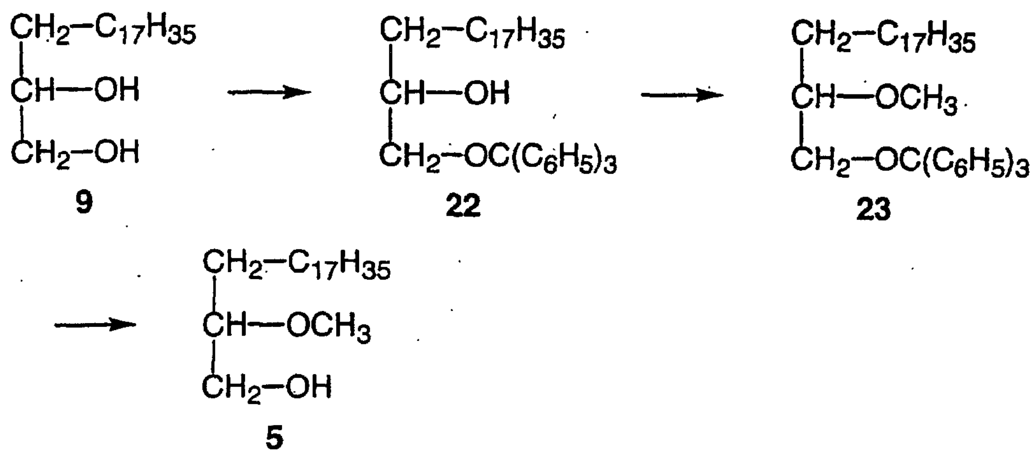

9 22 23 5

(1) Synthesis of Compound 22

To a solution of Compound 9(3.00 g, 9.54 mmol) in toluene(30 ml) were added pyridine(0.85 ml, 10.49 mmol) and trityl chloride(2.66 g, 9.54 mmol) and then stirred for 17 hours at 115°C. The reaction mixture was diluted with ethyl acetate, washed with 1N hydrochloric acid, water and saturated brine, successively, dried over sodium sulfate, and then concentrated. The residue was purified by silica gel column chromatography(silica gel 50 g, hexane:ethyl acetate=2:1 - chloroform), to give Compound 22(2.11 g, 40%) as a white wax.

$^1$H-NMR ($CDCl_3$) δ : 0.88 (3H, t, J=6.8 Hz), 1.26 (32H, m), 1.37 (2H, m), 2.28 (1H, d, J=3.4 Hz), 3.02 (1H, dd, J=7.8, 9.3 Hz), 3.19 (1H, dd, J=2.9, 9.3.Hz), 3.76 (1H, m), 7.15-7.35 (9H, m), 7.35 - 7.55 (6H, m).

(2) Synthesis of Compound 23

To a solution of Compound 22(2.01 g, 3.61 mmol) in N,N-dimethylformamide(12 ml) was added sodium hydride(60% content in oil)(0.22 g, 5.41 mmol) and stirred for an hour while being cooled with ice. Methyl iodide(0.34 ml, 5.41 mmol) was added to the mixture and stirred for 5 hours at room temperature. The reaction mixture was diluted with water and then concentrated. The obtained residue was diluted with ethyl acetate, washed with water, dried over sodium sulfate, and then concentrated. The residue was purified by silica gel column chromatography(silica gel 50 g, hexane - hexane:ethyl acetate=20:1), to give Compound 23 (2.06 g, 100%) as a colorless oil.

$^1$H-NMR ($CDCl_3$) δ : 0.88 (3H, t, J=6.8 Hz), 1.26 (32H, m), 1.49 (2H, m), 3.10 - 3.20 (2H, m), 3.27 (1H, m) , 3.41 (3H, s), 7.15 - 7.35 (9H, m), 7.35 - 7.55 (6H, m).

(3) Synthesis of Compound 5

To Compound 23(1.98 g, 3.47 mmol) were added triethyl borate(4 ml) and boric acid(2.14 g, 34.70 mmol) and stirred for 3 hours at 120°C. The reaction mixture was diluted with water, extracted with chloroform, dried over sodium sulfate, and then concentrated. The residue was purified by silica gel column chromatography(silica gel 50 g, chloroform-chloroform:methanol=50:1), to give Compound 5(0.91 g, 80%) as a white wax.

$^1$H-NMR ($CDCl_3$) δ : 0.88 (3H, t, J=6.8 Hz), 1.10 - 1.50 (33H, m), 1.50 - 1.65 (1H, m), 1.92 (1H, dd, J=4.9, 7.3 Hz), 3.26 (1H, ddd, J=3.4, 6.4, 9.3 Hz), 3.41 (3H, s), 3.48 (1H, ddd, J=4.9, 6.4, 11.7 Hz), 3.68 (1H, ddd, J=3.4, 7.3, 11.7 Hz).

Synthetic example 6 Synthesis of Compound 6

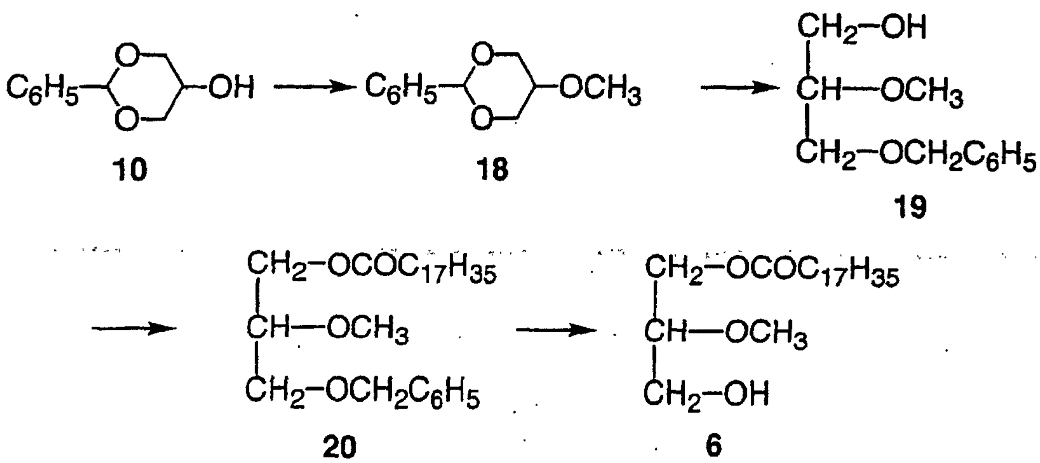

10 18 19 20 6

(1) Synthesis of Compound 18

To a solution of Compound 10(2.34 g, 13.00 mmol) in N,N-dimethylformamide(24 ml) was added sodium hydride(60% content in oil)(0.57 g, 14.30 mmol) and stirred for 1 hour while being cooled with ice. Methyl iodide(2.22 g, 15.64 mmol) was added to the mixture and stirred for 16.5 hours at room temperature. The reaction mixture was diluted with water, extracted with hexane and ethyl acetate, washed with saturated brine, dried over sodium sulfate, and then concentrated. The residue was purified by silica gel column chromatography(silica gel 40 g, hexane - hexane: ethyl acetate=10:1), to give Compound 18(0.90 g, 36%) as a colorless oil.

$^1$H-NMR ($CDCl_3$) δ : 3.42 (3H, s), 3.59 (3H, m), 4.41 (2H, m), 5.39 (1H, s), 7.30 - 7.40 (3H, m), 7.45- 7.50 (2H, m).

(2) Synthesis of Compound 19

To a solution of Compound 18(0.85 g, 4.39 mmol) in toluene(4 ml) was added 1.0M diisobutylaluminum hydride (DIBAL-H) in toluene(11.0 ml, 10.97 mmol) in argon atmosphere while being cooled with ice and stirred for 18 hours at room temperature. After methanol was added to the reaction mixture while being cooled with ice, 10% sodium hydroxide and water were further added thereto. The mixture was extracted with ethyl acetate, washed with saturated brine, dried over sodium sulfate, and then concentrated. The residue was purified by silica gel column chromatography (silica gel 25 g, hexane:ethyl acetate=5:1 - 2:1 - 1:1), to give Compound 19(0.82 g, 95%) as a colorless oil.

$^1$H-NMR ($CDCl_3$) δ : 2.15 (1H, br t), 3.40 - 3.50 (1H,m), 3.46 (3H, s), 3.50 - 3.70 (3H, m), 3.70 - 3.80 (1H, m), 4.55 (2H, s), 7.25 - 7.40 (5H, m).

(3) Synthesis of Compound 20

To a solution of Compound 19(0.77 g, 3.92 mmol) in toluene(8 ml) were added pyridine(0.34 g, 4.31 mmol) and a solution of stearoyl chloride(1.25 g, 4.12 mmol) in toluene(10 ml) in argon atmosphere while being cooled with ice. The mixture was stirred for 14 hours at room temperature, and further stirred for 2.5 hours at 50°C. After the insoluble matters were filtrated out, the filtrate was washed with saturated brine, dried over sodium sulfate, and then concentrated. The residue was purified by silica gel column chromatography(silica gel 50 g, hexane:ethyl acetate=50: 1 - 20:1 - 10:1). The obtained crude product(1.70 g) was purified again by silica gel column chromatography(silica gel 80 g, chloroform), to give Compound 20 (1.53 g, 84%) as a colorless oil.

$^1$H-NMR ($CDCl_3$) δ : 0.88 (3H, t, J=6.8 Hz), 1.25 (28H, m), 1.60 (2H, m), 2.30 (2H, t, J=7.6 Hz), 3.45 (3H, s), 3.50 - 3.65 (3H, m), 3.60 (1H, dd, J=5.2,11.7 Hz), 4.27 (1H, dd, J=4.2,11.7 Hz), 4.55 (2H, s), 7.25 - 7.40 (5H, m).

(4) Synthesis of Compound 6

To a solution of Compound 20(1.50 g, 3.24 mmol) in tetrahydrofuran(15 ml) was added 10% palladium-carbon

(50% water content)(0.30 g) and stirred for 12.5 hours in hydrogen atmosphere at room temperature. After the insoluble matters were filtrated out, the filtrate was concentrated. The obtained residue was purified by silica gel column chromatography(silica gel 30 g, hexane:ethyl acetate=10:1 - 5:1), to give Compound 6(1.10 g, 91%) as a white wax.

$^1$H-NMR ($CDCl_3$) δ : 0.88 (3H, t, J=6.8 Hz), 1.26 (28H, m), 1.62 (2H, m), 2.12 (1H, t, J=6.4 Hz), 2.33 (2H, t, J=7.3 Hz), 3.47 (3H, s), 3.24 - 3.52 (1H,m), 3.56 - 3.65 (1H, m), 3.69 (1H, m), 4.20 (2H, d, J=4.9 Hz).

Synthetic example 7 Synthesis of Compound 7

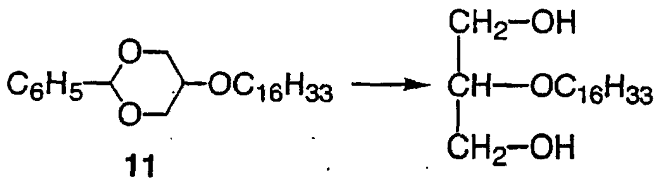

11

7

To a solution of Compound 11(1.30 g, 3.21 mmol) in tetrahydrofuran(13 ml) was added 10% palladium-carbon (50% water content)(0.26 g). The mixture was stirred for 24 hours in hydrogen atmosphere at room temperature, and further stirred for 8 hours at 50°C. After the insoluble matters were filtrated out, the filtrate was concentrated. The obtaned residue was purified by silica gel column chromatography(silica gel 40 g, chloroform-chloroform:methanol=50: 1), to give Compound 7(0.83 g, 82%) as a white solid.

$^1$H-NMR ($CDCl_3$) δ : 0.88 (3H, t, J=6.8 Hz), 1.26 (26H, m), 1.59 (2H, m), 2.37 (2H, br s), 3.44 (1H, m), 3.56 (2H, t, J=6.8 Hz), 3.69 (2H, m), 3.74 (2H,m).

Synthetic example 8 Synthesis of Compound 8

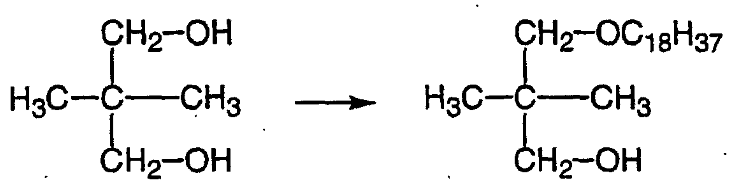

24

8

To a solution of Compound 24(2.50 g, 24.00 mmol) in N,N-dimethylformamide(51 ml) was added sodium hydride(60% content in oil)(2.11 g, 52.75 mmol) and stirred for an hour while being cooled with ice. Then, the mixture, with 1-bromooctadecane(9.93 g, 29.78 mmol) added thereto, was stirred for 16.5 hours at room temperature. The reaction mixture was diluted with water, extracted with hexane and ethyl acetate, washed with saturated brine dried over sodium sulfate, and then concentrated. The residue was purified by silica ge column chromatography(silica gel 275 g, hexane - hexane:ethyl acetate=20:1), to give Compound 8(3.32 g, 39%) as a white solid.

$^1$H-NMR ($CDCl_3$) δ : 0.88 (3H, t, J=6.8 Hz), 0.92 (6H, s), 1.26 (30H, m), 1.56 (2H, m) 2.91 (1H, t, J=5.9 Hz), 3.27 (2H, s), 3.40 (2H, t, J=6.8 Hz), 3.45 (2H, d, J=5.9 Hz).

Synthetic example 9 Synthesis of Compound 9

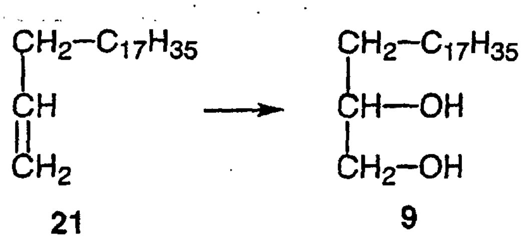

To a solution of Compound 21(5.00 g, 17.82 mmol) in dichloromethane(20 ml) was added m-chloroperbenzoic acid(80%)(4.61 g, 21.38 mmol) and stirred for 5 hours at 45°C. After 10% sodium hydrogensulfite(3 ml) was added, the reaction mixture was diluted with chloroform. The insoluble matters were filtrated out and the filtrate was concentrated. To a solution of the obtained residue(8.55 g) in dioxane(20 ml) and water(7 ml) was added concentrated sulfuric acid(6 drops) and stirred for 2 hours at 100°C. The reaction mixture was concentrated and the residue was purified by silica gel column chromatography(silica gel 100 g, chloroform - chloroform:methanol=20:1), to give Compound 9(3.79 g, 68%) as a white solid.

$^1$H-NMR ($CDCl_3$) δ : 0.88 (3H, t, J=6.8 Hz), 1.26 (32H, m), 1.44 (2H, m), 1.91 (1H, br s), 2.02 (1H, br s), 3.44 (1H, dd, J=7.6,11.0 Hz), 3.66 (1H, dd, J=2.9,11.0 Hz), 3.71 (1H, m).

Compound(I) of the present invention has a hair anagen stage prolonging effect by means of preserving or promoting a multiplicative growth activity of the human trichoepithelial cell and a human hair shaft elongation activity. Therefore, it is useful for an effective ingredient contained in a hair growth promoting composition(which is a general idea including hair growth composition, hair regrowth promoting composition, hair anagen stage prolonging composition and the like), which purpose is to promote hair regrowth and to prevent hair loss in human. By applying it on scalp, care, improvement, or prevention of hair loss can be expected.

The hair growth promoting composition of the present invention can apply to pathological alopecia such as alopecia areata, alopecia pityrodes or alopecia seborrheica in addition to thin hair or hair loss what is called male pattern baldness or androgenic alopecia. It can be considered that the present composition is effective especially to hair loss caused by relative decrease in a rate of hair at hair anagen stage with respect to telogen hair due to a hair anagen stage period shortened by inactive growth of the trichoepithelial cell nearby hair root and so on.

The hair growth promoting composition of the present invention can be manufactured by a normal way according to its pharmaceutical form. The pharmaceutical form, which is not particularly limited and can be determined freely as long as the effect of the present invention can be exerted, may be a solution form such as a tonic, an emulsion form such as a milky lotion or a cream, a ointment, a suspension, a gel, an aerosol, and a mousse. The product form can be also selected freely from a drug, quasi-drug or cosmetic for hair care which purpose is hair growth effect such as hair loss prevention, hair regrowth, or hair growth. For example, it may be a hair regrowth promoting composition, a hair growth composition, a scalp treatment, a hair tonic, a shampoo, a rinse, a hair pack, a lotion, a conditioner, and a scalp treatment.

In the hair growth promoting composition of the present invention, Compound (I) is 0.0005 - 20 % by weight, preferably 0.01 - 5 % by weight, based on the entire composition,. Less than 0.0005 % by weight may lead to an insufficient effect of the present invention, while more than 20% by weight may lead to a pharmaceutically unfavorable composition. The hair growth promoting composition of the present invention can comprise two or more of Compound(I).

The hair growth promoting composition of the present invention is percutaneously administrated by directly applying or spraying on skin. The best dosage thereof must be determined suitably according to age, individual difference, symptom and the like. Usually, in human, 0.01-100 mg/kg, preferably 0.1 to 10 mg/kg is administrated per day in 1-4 doses.

The hair growth promoting composition of the present invention can be prepared by appropriately incorporating, in addition to the aforementioned essential ingredient, other ingredients normally used in a cosmetic, quasi-drug or drug, as the occasion demands. Examples of such ingredients include powders, liquid fats or oils, solid fats or oils, waxes, hydrocarbons, higher fatty acids, higher alcohols, esters, silicones, anionic surfactants, cationic surfactants, amphoteric surfactants, nonionic surfactants, humectants, water-soluble polymers, thickeners, film-forming agents, ultraviolet absorbing agents, metal ion sequestering agents, lower alcohols, multivalent alcohols, sugars, amino acids,

organic amines, synthetic resin emulsions, pH adjusting agents, skin nutrients, vitamins, antioxidants, antioxidant aids, perfumes, water, and the like.

Examples of powders include inorganic powders such as talc, kaolin, mica, sericite, muscovite, phlogopite, synthetic mica, red mica, biotite, lithia mica, venniculite, magnesium carbonate, calcium carbonate, aluminum silicate, valium silicate, calcium silicate, magnesium silicate, strontium silicate, tungsten acid metal salt, magnesium, silica, zeolite, barium sulfate, calcinated calcium sulfate (calcined gypsum), calcium phosphate, fluorine apatite, hydroxyapatite, ceramic powder, metal soap(zinc myristate, calcium palmitate and aluminum stearate) and boron nitride; organic powders such as polyamide resin powder (nylon powder), polyethylene powder, methyl polymethacrylate powder, polystyrene powder, resin powder of copolymer of styrene and acrylic acid, benzoguanamine resin powder, polyethylene tetrafluoride powder and cellulose powder; inorganic white pigments such as titanium dioxide and zinc oxide; inorganic red series pigments such as iron oxide (red iron oxide) and iron titanate; inorganic brown series pigments such as γ-iron oxide; inorganic yellow series pigments such as yellow iron oxide and loess; inorganic black series pigments such as black iron oxide, carbon black and lower titanium oxide; inorganic purple series pigments such as mangoviolet and cobaltviolet; inorganic green series pigments such as chromium oxide, chromium hydroxide and cobalt titanate; inorganic blue series pigments such as ultramarine and Prussian blue; pearl pigments such as titanium oxide-coated mica, titanium oxide-coated bismuth oxychloride, titanium oxide-coated talc, colored titanium oxide-coated mica, bismuth oxychloride and fish scale foil; metal powder pigments such as aluminum powder and copper powder; organic pigments such as Red No.201, Red No.202, Red No.204, Red No.205, Red No.220, Red No.226, Red No.228, Red No.405, Orange No.203, Orange No.204, Yellow No.205, Yellow No.401 and Blue No.404; organic aluminum lake pigments with zirconium, barium, aluminum and the like such as Red No.3, Red No.104, Red No.106, Red No.227, Red No.230, Red No.401, Red No.505, Orange No.205, Yellow No.4, Yellow No.5, Yellow No. 202, Yellow No.203, Green No.3 and Blue No.1; natural colors such as chlorophyll and β -carotene; and the like.

Examples of liquid fats or oils include avocado oil, camellia oil, turtle oil, macadamia nut oil, corn oil, mink oil, olive oil, rapeseed oil, yolk oil, sesame oil, persicary oil, wheet germ oil, sasanqua oil, castor oil, linseed oil, safflower oil, cottonseed oil, perilla oil, soybean oil, peanut oil, tea seed oil, kaya oil, rice bran oil, Chinese paulownia oil, Japanese paulownia oil, jojoba oil, germ oil, triglycerides, glyceryl trioctanoate, glyceryl triisopalmitate and the like.

Examples of solid fats or oils include cacao butter, coconut oil, horse fat, hydrogenated coconut oil, palm oil, beef tallow, mutton tallow, hardened beef tallow, palm kernel oil, lard, beef bone fat, Japan wax kernel oil, hardened oil, beef foot fat, Japan wax, hydrogenated castor oil and the like.

Examples of waxes include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether and the like.

Examples of the hydrocarbon oils include liquid paraffin, ozokerite, squalane, pristane, paraffin, ceresin, squalene, vaseline, microcrystalline wax and the like.

Examples of the higher fatty acid include lauric acid, myristic acid, palmitic acid, stearic acid, behenic acid, oleic acid, petroselinic acid, 12-hydroxystearic acid, undecylenic acid, tallic acid, isostearic acid, linoleic acid, linolenic acid, eicosapentaenoic acid (EPA), docosahexaenoic acid (DHA) and the like.

Examples of the higher alcohols include straight alcohols such as lauryl alcohol, cetyl alcohol, stearyl alcohol, behenyl alcohol, myristyl alcohol, oleyl alcohol and cetostearyl alcohol; branched alcohols such as monostearylglycerin ether (batyl alcohol), 2-decyltetradecinol, lanolin alcohol, cholesterol, phytosterol, hexyldodecanol, isostearyl alcohol and octyldodecanol; and the like.

Examples of the synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, monoisostearic acid N-alkyl glycol, neopentyl glycol dicaprate, diisostearyl malate, glyceryl di-2-heptylundecanoate, trimethylolpropane tri-2-ethylhexanoate, trimethylol propane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceryl tri-2-heptylundecanoate, castor oil fatty acid methyl ester, oleyl oleate, cetostearyl alcohol, acetoglyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, triethyl citrate and the like.

Examples of the silicone oils include chain polysiloxanes such as dimethylpolysiloxane, methylphenylpolysiloxane and methylhydrogenpolysiloxane; cyclic polysiloxanes such as decamethylpolysiloxane, dodecamethylpolysiloxane and tetramethylhydrogenpolysiloxane; silicone resins having a three dimensional network structure; silicone rubbers; and the like.

Examples of the anionic surfactants include fatty acid soaps such as soap base, sodium laurate and sodium palmitate; higher alkyl sulfates such as sodium lauryl sulfate and potassium lauryl sulfate; alkyl ether sulfates such as triethanolamine POE-lauryl sulfate and sodium POE lauryl sulfate; N-acylsarcosinic acids such as sodium lauroylsarcosinate; higher fatty acid amide sulfates such as sodium N-myristoyl-N-methyltaurate, sodium cocoyl methyltaurate and sodium lauroyl methyltaurate; phosphate salts such as sodium POE oleyl ether phosphate and POE stearyl ether phosphoric acid; sulfosuccinates such as sodium di-2-ethylhexylsulfosuccinate, sodium monolauroyl monoethanolamide polyoxyethylenesulfosuccinate and sodium lauryl polypropyleneglycol sulfosuccinate; alkylbenzenesulfonates such as sodium linear dodecylbenzenesulfonate, triethanolamine linear dodecylbenzenesulfonate and linear dodecylbenzenesulfonic acid; N-acylglutamates such as monosodium N-lauroylglutamate, disodium N-stearoylglutamate and monosodium N-myristoyl-L-glutamate; higher fatty acid ester sulfates such as sodium hydrogenated cocoglyceride sulfate; sulfated oils such as turkey red oil; POE alkyl ether carboxylic acids; POE alkyl allyl ether carboxylates; $\alpha$ -olefinsulfonates; higher fatty acid ester sulfonates; secondary alcohol sulfates; higher fatty acid alkylolamide sulfate; sodium lauroyl monoethanolamide succinate; ditriethanolamine N-palmitoylaspartate; sodium caseinate; and the like.

Examples of the cationic surfactants include alkyl trimethyl ammonium salts such as stearyl trimethyl ammonium chloride and lauryl trimethyl ammonium chloride; distearyl dimethyl ammonium chloride dialkyl dimethyl ammonium salts; poly(N,N'-dimethyl-3,5-methylenepiperidinium) chloride; alkyl pyridinium salts such as cetylpyridinium chloride; alkyl quaternary ammonium salts; alkyl dimethyl benzyl ammonium salts; alkylisoquinolinium salts; dialkylmorphonium salts; POE alkylamines; alkylamine salts; polyamine fatty acid derivatives; amyl alcohol fatty acid derivatives; benzalkonium chloride; benzetonium chloride; and the like.

Examples of the amphoteric surfactants include imidazoline series amphoteric surfactants such as 2-undecyl-N,N,N-(hydroxyethylcarboxymethyl)-2-imidazoline sodium salt and 2-cocoyl-2-imidazolinium hydroxide-1-carboxyethyloxy disodium salt; betaine series surfactants such as 2-heptadecyl-N-carboxymethyl-N-hydroxyethylimidazolinium betaine, lauryldimethylamino acetic acid betaine, alkylbetaines, amidebetaines and sulfobetaines; and the like.

Examples of the lipophilic nonionic surfactants include sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, penta-2-ethylhexylic acid diglycerol sorbitan and tetra-2-ethylhexylic acid diglycerol sorbitan; glycerin polyglycerin fatty acid esters such as mono-cottonseed oil fatty acid glycerin ester, glyceryl monoerucate, glyceryl sesquioleate, glyceryl monostearate, glyceryl $\alpha$, $\alpha$ ' -oleate pyroglutamate and glyceryl monostearate malate; propylene glycol fatty acid esters such as propylene glycol monostearate; hydrogenated castor oil derivatives; glyceryl alkyl ethers; and the like.

Examples of the hydrophilic nonionic surfactants include POE-sorbitan fatty acid esters such as POE-sorbitan monooleate, POE-sorbitan monostearate, POE-sorbitan monooleate and POE-sorbitan tetraoleate; POE-sorbit fatty acid esters such as POE-sorbit monolaurate, POE-sorbit monooleate, POE-sorbit pentaoleate and POE-sorbit monostearate; POE-glycerin fatty acid esters such as POE-glyceryl monostearate, POE-glyceryl monoisostearate, and POE-glyceryl triisostearate; POE-fatty acid esters such as POE-monooleate, POE-distearate, POE-monodioleate and ethylene glycol distearate; POE-alkyl ethers such as POE-lauryl ether, POE-oleyl ether, POE-stearyl ether, POE-behenyl ether, POE-2-octyldodecyl ether and POE-cholestanol ether; POE alkyl phenyl ethers such as POE-octyl phenyl ether, POE-nonyl phenyl ether and POE-dinonyl phenyl ether; Pluronic type surfactants such as Pluronic; POE•POP-alkyl ethers such as POE•POP-cetyl ether, POE•POP-2-decyltetradecyl ether, POE•POP-monobutyl ether,POE•POP-hydrogenated lanolin and POE•POP-glyceryl ether, fused tetraPOE•tetraPOP-ethylenediamines such as Tetronic; POE-castor oil hydrogenated castor oil derivatives such as POE-castor oil, POE-hydrogenated castor oil, POE-hydrogenated castor oil monoisostearate, POE-hydrogenated castor oil triisostearate, POE-hydrogenated castor oil monopyroglutamic acid monoisostearic acid diester and POE-hydrogenated castor oil maleate; POE-beeswax• lanolin derivatives such as POE-sorbit beeswax; alkanolamides such as coconut oil fatty acid diethanolamide, lauric acid monoethanolamide and fatty acid isopropanolamide; POE-propylene glycol fatty acid esters; POE-alkylamines; POE-fatty acid amides; sucrose fatty acid esters; POE-nonyl phenyl formamide condensates; alkylethoxydimethylamine oxides; trioleylphosphoric acid; and the like.

Examples of the humectants include polyethylene glycol, propylene glycol, glycerin, 1,3-butylene glycol, xylitol, sorbitol, maltitol, chondroitin sulfate, hyaluronic acid, mucoitin sulfate, charonin acid, atellocollagen, cholesteryl-12-hydroxystearate, sodium lactate, bile salt, dl-pyrrolidone carboxylate, short chain soluble collagen, diglycerin(EO) PO adducts, the sixteen night rose extract, yarrow extract, melirote extract and the like.

Examples of the natural water-soluble polymers include plant series polymers such as gum arabic, tragacanth gum, galactan, guar gum, carob gum, karaya gum, carrageenan, pectin, agar, quinceseed (Cydonia oblonga), Alga colloid (brown alga extract), starch (rice, corn, potato, wheat) and glycyrrhizic acid; microorganizm series polymers such as xanthan gum, dextran, succinoglucan and pullulan; animal series polymers such as collagen, casein, albumin, gelatin; and the like.

Examples of the semi-synthetic water-soluble polymers include starch series polymers such as carboxymethylstarch and methylhydroxypropylstarch; cellulose series polymers such as methylcellulose, ethylcellulose, methylhydroxypropylcellulose, hydroxyethylcellulose, cellulose sodium sulfate, hydroxypropylcellulose, carboxymethylcellulose, sodium carboxymethylcellulose, crystalline cellulose and cellulose powder; alginic acid series polymers such as sodium alginate and propylane glycol alginate; and the like.

Examples of the synthetic water-soluble polymers include vinyl series polymers such as polyvinyl alcohol, polyvinyl methyl ether, polyvinyl pyrrolidone and carboxyvinyl polymer (Carbopol); polyoxyethylene series polymers such as polyethylene glycol 20,000, 40,000 and 60,000; polyoxyethylene polyoxypropylene copolymers; acrylic series polymers such as sodium polyacrylate, ethyl polyacrylate and polyacrylamide; polyethyleneimines; cationic polymers; and the like.

Examples of the inorganic water-soluble polymers include bentonite, aluminum magnesium silicate (Veegum), laponite, hectorite, silicic anhydride and the like.

Examples of the thickeners include gum arabic, carrageenan, karaya gum, tragacanth gum, carob gum, quinceseed (Cydonia oblonga), caseine, dextrin, gelatin, sodium pectinate, sodium alginate, methylcellulose, ethylcellulose, CMC, hydroxyethylcellulose, hydroxypropylcellulose, PVA, PVM, PVP, sodium polyacrylate, carboxyvinyl polymer, locust bean gum, guar gum, tamarind gum, dialkyldimethylammonium sulfate cellulose, xanthan gum, aluminium magnesium silicate, bentonite, hectorite and the like.

Examples of the ultraviolet absorbing agents include benzoic acid series ultraviolet absorbing agents such as paraaminobenzoic acid (hereinafter referred to as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester and N,N-dimethyl PABA ethyl ester; anthranilic acid series ultraviolet absorbing agents such as homomenthyl-N-acetyl anthranilate; salicylic acid series ultraviolet absorbing agents such as amyl salicylate, menthyl salicylate, homomenthyl salicylate, octyl salicylate, phenyl salicylate, benzyl salicylate and p-isopropanol phenyl salicylate; cinnamic acid series ultraviolet absorbing agents such as octyl cinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate(2-ethylhexyl p-methoxycinnamate), 2-ethoxyethyl p-methoxycinnamate, cyclohexyl p-methoxycinnamate, ethyl $\alpha$ -cyano- $\beta$ -phenylcinnamate, 2-ethylhexyl $\alpha$ -cyano- $\beta$ -phenylcinnamate and mono-2-ethylhexanoylglyceryl diparamethoxycinnamate; benzophenone series ultraviolet absorbing agents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone and 4-hydroxy-3-carboxybenzophenone; 3 -(4'-methylbenzylidene)-d,1-camphor, 3-benzylidene-d,1-camphor; urocanic acid; ethyl urocanate; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzaladine; dianisoylmethane; 4-methoxy-4'-t-butyldibenzoylmethane; 5-(3,3-dimethyl-2-norbornylidene) -3-pentane-2-one; and the like.

Examples of the sequestering agents include 1-hydroxyethane-1,1-diphosphonic acid, tetrasodium 1-hydroxyethane- 1,1 -diphosphonate, disodium edetate, trisodium edetate, tetrasodium edetate, sodium citrate, sodium polyphosphate, sodium methaphosphate, gluconic acid, phosphoric acid, citric acid, ascorbic acid, succinic acid, edetic acid and the like.

Examples of the lower alcohol include methanol, ethanol, propanol, isopropanol, isobutyl alcohol, t-butyl alcohol and the like.

Examples of the polyols include diols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol and octylene glycol; triols such as glycerin, trimethylolpropane and 1,2,6-hexanetriol; tetraols such as pentaerythritol; pentaols such as xylitol; hexaols such as sorbitol and mannitol; polyol polymers such as diethylene glycol, dipropylene glycol, triethylene glycol, polypropylene glycol, tetraethylene glycol, diglycerin, polyethylene glycol, triglycerin, tetraglycerin and polyglycerin; diol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether and ethylene glycol dibutyl ether; diol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether and dipropylene glycol butyl ether; diol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate and propylene glycol monophenyl

ether acetate; glycerin monoalkyl ethers such as xyl alcohol, selachyl alcohol and batyl alcohol; suger alcohols such as sorbitol, maltitol, maltotriose, mannitol, sucrose, erythritol, glucose, fructose, starch-degraded suger, maltose, xylulose and starch-degraded suger reduced alcohol; glysolid; tetrahydrofurfuryl alcohol; POE-tetrahydrofurfuryl alcohol; POP-butyl ether; POP•POE-butyl ether; tripolyoxypropylene glyceryl ether; POP-glyceryl ether; POP-glyceryl ether phosphoric acid; POP•POE-pentaerythritol ether; polyglycerin; and the like.

Examples of the monosaccharides include trioses such as D-glycerylaldehyde, dihydroxyacetone; tetroses such as D-erythrose, D-erythrulose, D-threose and erythritol; pentoses such as L-arabinose, D-xylose, L-lyxose, D-arabinose, D-ribose, D-ribulose, D-xylulose and L-xylulose; hexoses such as D-glucose, D-talose, D-psicose, D-galactose, D-fructose, L-galactose, L-mannose and D-tagatose; heptoses such as aldoheptose and heprose; octoses such as octulose; deoxysugars such as 2-deoxy-D-ribose, 6-deoxy-L-galactose and 6-deoxy-L-mannose; aminosuger such as D-glucosamine, D-galactosamine, sialic acid, aminouronic acid and muramic acid; uronic acids such as D-glucronic acid, D-mannuronic acid, L-guluronic acid, D-galacturonic acid and L-iduronic acid; and the like.

Examples of the oligosaccharides include sucrose, guntianose, umbelliforose, lactose, planteose, isoliqunoses, $\alpha$ , $\alpha$ -trehalose, raffinose, liqunoses, umbilisine, stachyose belbascoses and the like.

Examples of the polysaccharides include cellulose, quinceseed, chondroitin sulfate, starch, galactan, dermatan sulfate, glycogen, gum arabic, heparan sulfate, hyaluronic acid, tragacanth gum, keratan sulfate, chondroitin, xanthan gum, mucoitin sulfate, guar gum, dextran, kerato sulfate, locust bean gum, succinoglucan, charonic acid and the like.

Examples of the amino acids include neutral amino acids such as glycine, alanine, valine, leucine, isoleucine, serine, threonine, tryptophan, cystine, cysteine, methionine, proline and hydroxyproline; acidic amino acids such as aspartic acid, glutamic acid, asparagine and glutamine; and basic amino acids such as arginine, histidine, lysin and hydroxylysine; and the like. In addition, examples of the amino acid derivatives include sodium acylsarcosinate (sodium lauroylsarcosinate), acylglutamiate, sodium acyl $\beta$ -alanate, glutathione, pyrrolidinecarboxylic acid and the like.

Examples of the organic amines include monoethanolamine, diethanolamine, triethanolamine, morpholine, triisopropanolamine, 2-amino-2-methyl-1,3-propanediol, 2-amino-2-methyl-1-propanol and the like.

Examples of the synthetic resin emulsions include acrylic resin emulsion, ethyl polyacrylate emulsion, acrylic resin solution, alkyl polyacrylate emulsion, polyvinyl acetate resin emulsion and the like.

Examples of the pH adjusting agents include buffers such as lactic acid-sodium lactate, citric acid-sodium citrate and the like.

Examples of the vitamins include vitamins A, $B_1$, $B_2$, $B_6$ and E and derivatives thereof, pantothenic acid and derivatives thereof, biotinic acid and the like.

Examples of the antioxidants include tocopherols, dibutylhydroxytoluene, butylhydroxyanisole, gallic acid esters and the like.

Examples of the antioxidant aids include phosphoric acid, citric acid, ascorbic acid, maleic acid, malonic acid, succinic acid, fumaric acid, cephalin, hexamethaphosphate, phytic acid, ethylenediamine tetraacetic acid and the like.

Although the present invention will be explained by using specific examples in below, it should not be restricted thereto. The compounding amount and concentration were shown by weight % when there was no description.

Experiment 1 Hair Regrowth Effect Test

The Compounds 1 to 5 described above were used as test materials. Each test material was dissolved in 70 % ethanol aqueous solution and the solution was subjected to the application as a sample(test material concentration: 50nM and 100nM). 70% Ethanol was also used as a control sample.

The hair regrowth effect test was performed using C3H/He mice, whose hair cycle was at the telogen stage according to the method of Ogawa et. al. (Normal and Abnormal Epidermal Differentiation, Edited by M. Seiji and I. A. Bernstein, Pages 159-170, 1982, Todai Shuppan).

Namely, 6 mice were used in a group and their hair of the regions of back was shaved by hair clippers. 0.1 ml of sample was applied on the shaved regions once a day. After 24 days, the hair regrowth area was measured and the hair regrowth area rate was calculated based on the hair regrowth area with respect to the shaved area. The results are shown in Table 1 (expressed by average values).

Table 1

| Test material | Hair regrowth area rate | |
|---|---|---|
| | 50nM | 100nM |
| Compound 1 | 70% | 93% |
| Compound 2 | 65% | 88% |

Table 1 (continued)

| Test material | Hair regrowth area rate | |
|---|---|---|
| | 50nM | 100nM |
| Compound 3 | 15% | 74% |
| Compound 4 | 68% | 78% |
| Compound 5 | 70% | 76% |
| Control(70% Ethanol) | 2% | |

As evident form Table 1, in hair regrowth test in mice, compounds of the present invention have significant hair regrowth effects as compared with the control sample.

Experiment 2 Trichoepithelial Cell Growth Promoting Effect Test

According to the method described in JP-A-2000-63242, a cultured trichoepithelial cell of a human scalp was used to measure a trichoepithelial cell growth promoting effect.

1. Collection of human trichoepithelial cell

From a human scalp obtained as a by-product of a surgical operation, a hair follicle at a anagen stage in a hair cycle was isolated mechanically under the observation by a stereoscopic microscope. This hair follicle at a anagen stage was treated in a Dulbecco's modified MEM(DMEM) supplemented with 1000 U/ml dispase and 0.2 % collagenase for 30 minutes at 37°C. Adipose tissues, connective tissue root sheath and dermal papilla were removed therefrom using the tip of a syringe needle and the hair follicle was treated in a phosphate buffer containing 0.05% EDTA for 5 minutes at 37°C.

Subsequently, the hair follicle was placed on a culture dish coated with Type I collagen, and incubated in a KGM medium (serum-free keratinocyte growth medium). After incubation for 4 to 5 days, the culture medium was exchanged upon ensuring the adhesion of the hair follicle on the bottom face of the culture dish and the growth of the trichoepithelial cell, and thereafter the culture medium was exchanged every 2 days.

After the cell thus proliferated was treated in a phosphate buffer containing 0.05% trypsin and 0.02% EDTA for 5 minutes at 37°C, the reaction was quenched using an equal volume of 0.1% trypsin inhibitor and the cell was recovered by centrifugation (800 x g, 5 minutes).

Subsequently, the cell was suspended in the serum-free culture medium described above, and inoculated at the density of 5000 cells/$cm^2$ onto a culture dish coated with Type I collagen. The culture medium was exchanged every 2 days until the cell became subconfluent. After treating the cell again in the phosphate buffer containing 0.05% trypsin and 0.02% EDTA for 5 minutes at 37°C, the reaction was quenched using an equal volume of 0.1% trypsin inhibitor and the cell was recovered by centrifugation (800 x g, 5 minutes). The human trichoepithelial cell thus obtained was adjusted at the concentration of 100,000 cells/ml with a cell cryopreservation solution (Cellbanker: DIA-IATRON) added thereto, and dispensed into each freezing tube as a 100,000 cells(lml), which was then stored as being frozen. The cell counts described above were calculated using a hemocytometer.

2. Measurement of trichoepithelial cell growth promoting effect of target material

A. Preparation of trichoepithelial cell

After inoculating the trichoepithelial cell obtained in the step described above into a culture flask, the cell was treated with a phosphate buffer containing 0.25% trypsin and 0.02% EDTA. The reaction was quenched with 0.1% trypsin inhibitor, and then the mixture was centrifuged at 1,500 rpm for 5 minutes. The supernatant was removed, and the precipitated cell pellet was combined with a KGM medium to prepare a cell suspension.

The cell suspension in the KGM medium was inoculated to a Type I collagen-coated 96-well microplate(Falcon) at 3,000 cells(0.2 ml)/well and allowed to stand for about 20 minutes at room temperature until the cell went down onto the bottom. Then, the cell was cultured for 1 day in an incubator in the presence of 5% $CO_2$ at 37°C, whereby obtaining an intended cultured human trichoepithelial cell.

B. Preparation of test medium

(1) Preparation of test material-supplemented medium

A test material and DMSO were added to a KGM medium at the final concentrations of 1.0 x $10^{-6}$ mol/L and 0.1 %, respectively.

(2) Preparation of control medium

Negative control: DMSO was added to a KGM medium at the final concentration of 0.1 %.

Positive control: Insulin and hydrocortisone were dissolved in DMSO, which was then added to a KGM medium. The final concentrations of insulin, hydrocortisone and DMSO were 5 µg/ml, 0.5 µg/ml and 0.1%, respectively.

C. Exchange with target material medium

The KMG medium in the 96-well microplate employed for preparing the cultured human trichoepithelial cell in Step A described above was exchanged with a test material-supplemented medium or a control medium (200 µl/well), and the incubation was continued for 2 days in the presence of 5% $CO_2$ at 37°C.

D. Cell growth measurement

A 1/10 volume, based on the medium, of Alamer Blue(Alamer Bioscience Inc.) was added and the incubation was conducted for 6 hours at 37°C (5% $CO_2$).

After incubation, the reaction system was examined for the absorbances at 595 nm and 570 nm using a microplate reader(BioRad) to measure a Alamer Blue reduction rate, and a cell growth degree ($A_2$) of the test material-supplemented medium was calculated in accordance with the following equation.

$$\text{Cell growth degree } (A_2) \text{ in presence of test material} = (A_1/N_1) \times 100\ (\%)$$

$A_1$ : Alamer Blue reduction rate of test material-supplemented medium
$N_1$: Alamer Blue reduction rate of negative control

A cell growth promoting index was further calculated from the cell growth degree in accordance with the following equation.

Cell growth promoting index of test material = $(A_2-N_2)/(P_2-N_2)$

$A_2$: Cell growth degree in presence of test sample
$N_2$: Cell growth degree of negative control
$P_2$: Cell growth degree of positive control

The cell growth promoting index of the negative control here becomes 0, while that of the positive control becomes 1.

In the cell growth degree equation indicated above, $N_2$ and $P_2$ are calculated by replacing $A_1$ with $N_1$ and $P_1$ (Alamer Blue reduction rate of positive control), respectively.

E. Results

Table 2

| Tested material | Cell growth promoting index |
|---|---|
| Compound 1 | 1.3 |
| Compound 2 | 1.5 |
| Compound 3 | 1.4 |
| Compound 4 | 1.4 |

Table 2 (continued)

| Tested material | Cell growth promoting index |
|---|---|
| Compound 5 | 1.2 |
| Compound 6 | 1.3 |
| Compound 7 | 1.5 |
| Compound 8 | 1.2 |
| Compound 9 | 1.2 |

As shown in Table 2, compounds of the present invention were proven to have a trichoepithelial cell growth activity, and also proven to have an ability of keeping and prolonging the hair anagen stage by means of preserving the multiplicative growth activity of the trichoepithelial cell.

Experiment 3 Hair regrowth inducing effect test by cell cluster-accompanied culture method

The hair regrowth inducing effect was evaluated using, as an index, an increase in respiration level of a cell cluster formed under the coexistence of a normal human outer root sheath cell and a normal human dermal papilla cell.

1. Cell preparation

Normal human dermal papilla cell (DP): A DP obtained by a primary culture from a head hair was subcultured three times in a 10% FBS-supplemented DMEM medium in a 75 $cm^2$ incubation flask coated with Type I collagen, and then stored while being frozen in a liquid nitrogen using a cell cryopreservation solution (Cellbanker II: DIA-IATRON).

Normal human outer root sheath cell (ORS): An ORS obtained by a primary culture from a head hair was subcultured three times in a SFM medium in a 75 $cm^2$ incubation flask coated with Type I collagen, and then stored while being frozen in a liquid nitrogen using Cellbanker II.

2. Cell incubation

The DP and ORS cells stored as frozen were thawed, washed with an ice-cooled SFM medium, dispersed in the same medium and subjected to an accurate cell density measurement using a hemocytometer. Each cell density was adjusted at $5 \times 10^4$ cells/ml accurately, and the cell dispersions in equal volumes were combined under cooling with ice. 80 µl aliquots of the combined cell suspending SFM medium were inoculated into individual wells of a 96-well microplate (Sumilon celltight, spheroid 96U plate), and incubated for 2 days. The cell count in a single well upon inoculation was 2,000 cells for each of DP and ORS, thus 4,000 cells in total.

3. Test material addition

Each 80 µl of a test material-supplemented William's E(+) medium (William's E medium supplemented with 10 µg/ml transferrin, 10 ng/ml hydrocortisone, 10 µg/ml insulin and 10 ng/ml sodium selenite) was added to each well and incubated further for 2 days.

The test material-supplemented William's E(+) medium added to each well was prepared by dissolving the test material in ethanol at a concentration of 10 mM or 100 mM and adding to William's E(+) medium at 0.2% each. Accordingly, the final concentration of the test material in each well was 10 µM or 100 µM, and each final concentration of ethanol was 0.1%.

As a negative control, 80 µl of William's E(+) medium containing 0.2% ethanol was added (final concentration of ethanol was 0.1 %).

4. Respiratory activity measurement

Alamer Blue, SFM medium and William's E(+) medium were mixed in 2:1:1 ratio, warmed at 37°C and then 40 µl aliquots were added to individual wells and allowed to react for 6 hours at 37°C. After completion of the reaction, 100 µl aliquots were taken from individual wells, and transferred to a white 96-well plate for fluorescence measurement (Opaque Plate).

A fluorometer (Labsystems Fluoroskan II) was used at the excitation wavelength of 544 nm and the fluorescent wavelength of 590 nm to measure the fluorescence intensity of each well, from which the fluorescent intensity of a cell-free reaction mixture (i.e., the reaction mixture treated similarly using a cell-free SFM medium) was subtracted to obtain

a respiration level under each condition. From thus obtained respiration level, a relative respiration level(%) was calculated according to the following equation.

$$\text{Relative respiration level(\%)}$$

$$=\{\text{Respiration level (test material)}\}/\{\text{Respiration level (negative control)}\} \times 100$$

The results are shown in Table 3.

Table 3

| | Relative respiration level (%) | |
|---|---|---|
| Test material | 10μM | 100μM |
| Compound 1 | 138.9 | 157.0 |
| Compound 2 | 108.5 | 122.3 |
| Compound 3 | 106.5 | 107.5 |
| Compound 4 | 126.8 | 133.6 |
| Compound 5 | 124.4 | 130.5 |
| Compound 6 | 101.0 | 102.0 |
| Compound 7 | 122.4 | 129.0 |
| Compound 8 | 105.6 | 111.9 |
| Compound 9 | 129.7 | 132.2 |

As evident from Table 3, compounds of the present invention gave an increase in the respiration level of a cell cluster formed under the coexistence of human DP and ORS, which reflects the activation of the hair follicle cell in the stage of transferring to a anagen stage. Therefore, it has become clear that those have a hair regrowth inducing effect.

Experiment 4 Investigation of hair shaft elongation

Compounds of the present invention were also examined for hair shaft elongation effect.

1. Organ culture of human hair follicle

A hair follicle in the anagen stage was isolated from a human scalp under a stereoscopic microscope observation, washed with "a medium prepared by adding penicillin, streptomycin and fungizone to Williams E medium (Gibco)" (hereinafter referred to as a (-) medium) and then examined for its length.

A medium obtained by supplementing the (-) medium further with 10 ng/ml hydrocortisone, 10 μg/ml insulin, 10 ng/ml sodium selenite and 10 μg/ml transferring (hereinafter referred to as a (+) medium) was dispensed in an aliquot of 1 ml per well into a 24-well microplate. The hair follicle described above was precipitated therein and incubated overnight at 37°C in the presence of 5% $CO_2$ (preincubation).

After the preincubation, the length of each hair follicle was measured again, and the hair follicles exhibiting elongations of 0.25 mm or longer in the preincubation were selected and classified into groups each containing 9 hair follicles so that the degree of the elongation became uniform between groups. The elongation of the hair shaft in a hair follicle was determined by inspecting the microplate described above visually using an inverted microscope with a micrometer attached thereto.

2. Evaluation of test material

A. Test material-containing medium

A DMSO solution of a test material was added to a (-) medium for preparation. The final concentrations of the test material and DMSO were $1.0 \times 10^{-5}$ mol/L and 0.1%, respectively.

B. Negative control medium

Only DMSO was added to a (-) medium at the final concentration of 0.1% for preparation.

C. Culture medium exchange and measurement

The culture medium of the hair follicle groups described above was replaced each with the test material-containing medium or negative control medium, and the incubation was continued further for 5 days at 37°C in the presence of 5% $CO_2$. The length of each hair follicle after 5-day incubation was measured, and the means were compared with each other. The results are shown in Table 4.

Table 4

| Test material | Mean of hair shaft elongation(mm) |
|---|---|
| Compound 1 | 1.7 |
| Compound 2 | 1.5 |
| Compound 3 | 1.6 |
| Compound 4 | 1.9 |
| Compound 5 | 1.4 |
| Compound 6 | 1.7 |
| Compound 7 | 1.2 |
| Compound 8 | 1.7 |
| Compound 9 | 1.5 |
| Negative control | 0.6 |

These results revealed that the elongation of a hair shaft in a hair follicle was promoted and a hair anagen stage prolonging effect was exerted as a well-balanced effect in an organ of a hair follicle.

Experiment 5 Trichogram test

A trichogram test was also performed in humans as a practical use test of a hair growth promoting composition.

1. Sample solution preparation

As test materials, Compound 1 and Compound 2 were employed. Each test material was dissolved in 70% ethanol similarly to Experiment 1 to obtain a sample (test material concentration: 50 nM).

2. Experimental method

The hair roots of hairs pulled out before and after the use of each sample were examined by a microscope to determine the telogen hair root count on the basis of the hair root morphology, and the increase or decrease in the rate was employed as an index for evaluating the hair growth promoting effect. The telogen hair root is the root of a hair whose growth was discontinued, and it has been found that a human complaining of his hair loss has a higher rate of these telogen hair roots when compared with a normal human.

Specifically, each sample was applied onto the scalp of each of 10 male subjects twice a day in a 2 ml aliquot each time continuously for 6 months. 100 hairs per subject were pulled out immediately before the application and immediately after completion of 6-month application to count the telogen hair roots. The rate (%) of the number of the subjects whose telogen hair root after the application was decreased by 20% or more, changed by ±20% or increased by 20% or more with respect to that before the application was calculated. The results are shown in Table 5.

Table 5

| Test material | Rate of telogen hair root | | |
|---|---|---|---|
| | 20% or more decrease | ± 20% | 20% or more increse |
| Compound 1 | 70% | 30% | 0% |
| Compound 2 | 60% | 30% | 10% |

As evident from Table 5, the application of compounds of the present invention gave a significant reduction in the telogen hair roots also in the practical use test. Therefore, It can be understood that they are effective as a hair growth promoting composition.

In the following, examples of the present invention will be shown. All examples exhibited significant effects in hair regrowth effect test, hair anagen stage prolonging effect test and Trichogram test described in Experiment 1, 2 and 4, respectively.

Example 1 O/W hair growth promoting milky lotion

| | |
|---|---|
| (Phase A) | |
| Polyoxyethylene(60) hardened caster oil | 2.0 wt% |
| Glycerin | 10.0 |
| Compound 1 | 1.0 |
| Dipropylene glycol | 10.0 |
| 1,3-Butylene glycol | 5.0 |
| Polyethylene glycol 1500 | 5.0 |
| (Phase B) | |
| Cetyl isooctanoate | 10.0 |
| Squalane | 5.0 |
| Vaseline | 2.0 |
| Propylparaben | 2.0 |
| (Phase C) | |
| Carboxyvinyl polymer 1% aqueous solution | 30.0 |
| Sodium hexametaphosphate | 0.03 |
| Ion-exchange water | 8.35 |
| (Phase D) | |
| Potassium hydroxide | 0.12 |
| Ion-exchange water | Balance |

(Preparation method)

Phases A and B were heated and dissolved at 60°C, separately. Both were mixed and treated with a homomixer, thereby obtaining a gel. Subsequently, dissolved Phase C was added to this gel, and then dissolved Phase D was added finally. The mixture was emulsified by a homomixer to obtain an O/W hair growth promoting milky lotion.

Example 2 Hair growth promoting cream

| | |
|---|---|
| (Phase A) | |
| Liquid paraffin | 5.0 wt% |
| Cetostearyl alcohol | 5.5 |
| Glyceryl monostearate | 3.0 |
| Compound 2 | 3.0 |
| Propylparaben | 0.3 |
| Perfume | 0.1 |
| (Phase B) | |
| Vitamin E succinate | 5.0 |
| Glycerin | 8.0 |

(continued)

| (Phase B) | |
|---|---|
| Dipropylene glycol | 20.0 |
| Polyethylene glycol 4000 | 5.0 |
| Sodium dodecyl sulfate | 0.1 |
| Sodium hexametaphosphate | 0.005 |
| Ion-exchange water | 45.095 |

(Preparation method)

Phase A and B, which were heated and dissolved separately, were mixed and then emulsified by a homomixer to obtain a hair growth promoting cream.

Example 3

| | |
|---|---|
| Compound 3 | 0.2 wt% |
| Stearyldimethylamine oxide | 0.5 |
| Polyoxyethylene(40) hardened caster oil | 1.0 |
| 95% Ethanol | 54.0 |
| Ion-exchange water | Balance |

(Preparation method)

Ion-exchange water was added to 95% ethanol, and polyoxyethylene(40) hardened caster oil and stearyldimethylamine oxide were added thereto. Then, Compound 3 was further added and dissolved into the mixture with stirring.

Example 4

| | |
|---|---|
| Sodium N-cocolauryl- β -aminopropionate | 0.2 wt% |
| Compound 4 | 0.1 |
| Sodium dodecylbenzenesulfonate | 0.5 |
| Polyoxyethylene(40) hardened caster oil | 1.0 |
| 95% Ethanol | 54.0 |
| Ion-exchange water | Balance |

(Preparation method)

Ion-exchange water was added to 95% ethanol, and polyoxyethylene(40) hardened caster oil, sodium dodecylbenzenesulfonate and sodium N-cocolauryl- β -aminopropionate were further added thereto. Then, Compound 4 was added and dissolved into the mixture with stirring.

Example 5 Lotion

| (Phase A) | |
|---|---|
| Sorbitol | 3.0 wt% |
| Glycerin | 5.0 |
| Resorcin | 0.02 |
| Ion-exchange water | Balance |
| (Phase B) | |
| Compound 6 | 0.1 |
| Polyoxyethylene(60) hardened caster oil | 0.5 |
| 95% Ethanol | 20.5 |
| Perfume | Q.S. |

(Preparation method)

Ingredients of Phase A were mixed and dissolved. A mixed solution of Phase B was added to the mixture while being stirring to be a homogeneous solution to prepare a lotion.

Example 6 Cream

| (Phase A) | |
|---|---|
| Beeswax | 10.0 wt% |
| Paraffin wax | 6.0 |
| Lanolin | 3.0 |
| Isopropyl myristate | 6.0 |
| Squalane | 8.0 |
| Liquid paraffin | 26.0 |
| Polyoxyethylene sorbitan stearate | 2.0 |
| Sorbitan monostearate | 4.2 |
| Antiseptics | Q.S. |
| (Phase B) | |
| Propylene glycol | 2.0 |
| Polyoxyethylene(60) hardened caster oil | 1.0 |
| Compound 6 | 0.1 |
| Purified water | Balance |

(Preparation method)

Ingredients of Phase A were mixed and dissolved with heating at about 75°C. A mixed solution of Phase B heated at 75°C was added to Phase A while being stirring. The mixture was cooled to 45°C while being stirred, and then left as it was to obtain a cream.

Example 7 O/W hair growth promoting milky lotion

| | |
|---|---|
| (Phase A) | |
| Compound 5 | 0.01 wt% |
| Polyoxyethylene(60) hardened caster oil | 2.0 |
| Glycerin | 10.0 |
| Dipropylene glycol | 10.0 |
| 1,3-Butylene glycol | 5.0 |
| Polyethylene glycol (Molecular weight 1500) | 5.0 |
| (Phase B) | |
| Cetyl isooctanoate | 10.0 |
| Squalane | 5.0 |
| Vaseline | 2.0 |
| Propylparaben | 2.0 |
| (Phase C) | |
| 1% Carboxyvinyl polymer aqueous solution | 30.0 |
| Sodium hexametaphosphate | 0.03 |
| Ion-exchange water | 8.35 |
| (Phase D) | |
| Ion-exchange water | 4.5 |
| (Phase E) | |
| Potassium hydroxide | 0.12 |
| Ion-exchange water | Balance |

(Preparation method)

Phase A and B were heated and dissolved at 60°C, separately. Both were mixed and treated with a homomixer to obtain a gel. Phase D was gradually added to this gel and dispersed by a homomixer. Then, dissolved Phase C and dissolved Phase E were added to the dispersed mixture, successively and then was emulsified by a homomixer to obtain an O/W hair growth promoting milky lotion.

Example 8 Hair growth promoting cream

| | |
|---|---|
| (Phase A) | |
| Compound 7 | 1.0 wt% |
| Liquid paraffin | 5.0 |
| Cetostearyl alcohol | 5.5 |
| Glyceryl monostearate | 3.0 |
| EO(20) 2-octyldodecyl ether | 3.0 |
| Propylparaben | 0.3 |
| Perfume | 0.1 |

(continued)

| (Phase B) | |
|---|---|
| Glycerin | 8.0 |
| Dipropylene glycol | 20.0 |
| Polyethylene glycol (Molecular weight 4000) | 5.0 |
| Sodium hexametaphosphate | 0.005 |
| Ion-exchange water | Balance |

(Preparation method)

Phase A and B were heated and dissolved separately. Both were mixed and emulsified by a homomixer o obtain a hair growth promoting cream.

Example 9 Hair tonic

| | |
|---|---|
| Compound 8 | 10.0 wt% |
| Peppermint (1,3-butylene glycol solution) | 0.1 |
| N,N-Dimethyl-2-dodecylamine oxide | 1.0 |
| Hinokitiol | 1.0 |
| Vitamin $B_6$ | 0.2 |
| Vitamin E acetate | 0.02 |
| Menthol | 0.2 |
| Swertia herb extract | 1.0 |
| Salicylic acid | 0.1 |
| Rosae rugosae flos (ethanol extract) | 0.5 |
| Propylene glycol | 2.0 |
| Sodium hyaluronate | 0.01 |
| Polyoxyethylene( 10) monostearate | 2.0 |
| 75% Ethanol | Balance |

(Preparation method)

The ingredients described above were added to 75% ethanol, successively and then dissolved with stirring to obtain a hair tonic.

Example 10 Hair tonic

| | |
|---|---|
| Compound 9 | 10.0 wt% |
| Althea (ethanol extract) | 1.5 |
| Coix (ethanol extract) | 1.5 |
| N,N-Dimethyl-2-tetradecylamine oxide | 0.05 |
| Hinokitiol | 1.0 |

(continued)

| | |
|---|---|
| Vitamin $B_6$ | 0.2 |
| Vitamin E acetate | 0.02 |
| Menthol | 0.2 |
| Salicylic acid | 0.1 |
| Pueraria root (ethanol extract) | 0.5 |
| Propylene glycol | 0.01 |
| Sodium hyaluronate | 0.01 |
| Polyoxyethylene( 10) monostearate | 2.0 |
| 70% Ethanol | Balance |

(Preparation method)

Ingredients described above were added to 70% ethanol, successively and then dissolved with stirring to obtain a hair tonic.

Example 11 Aerosol hair growth promoting composition

| | |
|---|---|
| (Stock solution) | |
| Compound 1 | 0.6 wt% |
| 95% Ethanol | 50.0 |
| Glycyrrhetinic acid | 0.1 |
| Althea(ethanol extract) | 0.05 |
| Peppermint(ethanol extract) | 0.05 |
| Swertia herb extract | 0.1 |
| Sodium lauryl sulfate | 0.1 |
| N,N-Dihydroxymethyl-2-decylamine oxide | 0.2 |
| Polyoxyethylene(40) hardened caster oil | 0.5 |
| Lactic acid | Q.S. |
| Sodium Lactate | Q.S. |
| Perfume | Q.S. |
| Coloring agents | Q.S. |
| Purified water | Balance |
| (Filling formulation) | |
| Stock solution | 50.0 |
| Liquefied petroleum gas | 50.0 |

(Preparation method)

Ingredients of the stock solution were dissolved and filled into a can. After a valve was fit to the can, the gas was filled therein to obtain a hair growth promoting composition.

## Claims

1. A hair growth promoting composition comprising, as an effective ingredient, a compound expressed by the following Formula (I):

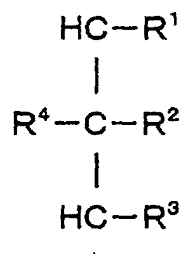

(I)

wherein one of $R^1$ to $R^4$ is selected from a group of $C_{14-22}$ alkyl, $C_{14-22}$ alkoxy and $C_{14-22}$ acyloxy groups, and the others are selected from a group of H, OH, $C_{1-3}$ alkyl and $C_{1-3}$ alkoxy groups;

when one of $R^1$ and $R^3$ is $C_{14-22}$ alkoxy group and one of $R^2$ and $R^4$ is $C_{1-3}$alkoxy group, the other of $R^1$ and $R^3$ is H, $C_{1-3}$ alkyl or $C_{1-3}$alkoxy group; and

when one of $R^1$ and $R^3$ is a $C_{14-22}$ acyloxy group, the compound of Formula (I) has at least one $C_{1-3}$alkoxy group therein.

2. The hair growth promoting composition according to Claim 1, wherein $R^1$ is $C_{14-22}$ alkyl group.

3. The hair growth promoting composition according to Claim 2, wherein $R^1$ is heptadecyl group and $R^2$ is methoxy group and $R^3$ is OH.

4. The hair growth promoting composition according to Claim 2, wherein $R^1$ is heptadecyl group and $R^2$ and $R^3$ are OH.

5. The hair growth promoting composition according to Claim 1, wherein $R^1$ is $C_{14-22}$ acyloxy group.

6. The hair growth promoting composition according to Claim 5, wherein $R^1$ is octadecanoyloxy group, $R^2$ is methoxy group and $R^3$ is OH.

7. The hair growth promoting composition according to Claim 1, wherein $R^2$ is $C_{14-22}$ alkoxy group.

8. The hair growth promoting composition according to Claim 7, wherein $R^2$ is hexadecyloxy group, $R^1$ is methoxy group and $R^3$ is OH.

9. The hair growth promoting composition according to Claim 7, wherein $R^2$ is hexadecyloxy group and $R^1$ and $R^3$ are OH.

10. The hair growth promoting composition according to Claim 1, wherein $R^1$ is $C_{14-22}$ alkoxy group.

11. The hair growth promoting composition according to Claim 10, wherein $R^1$ is hexadecyloxy group, and $R^2$ and $R^3$ are methoxy groups.

12. The hair growth promoting composition according to Claim 10, wherein $R^1$ is hexadecyloxy group and $R^2$ is OH and $R^3$ is methoxy group.

13. The hair growth promoting composition according to Claim 10, wherein $R^1$ is hexadecyloxy group, $R^2$ is H, and $R^3$ is OH.

14. The hair growth promoting composition according to any of Claims 1 to 13, wherein $R^4$ is H.

**15.** The hair growth promoting composition according to Claim 10, wherein $R^1$ is octadecyloxy group, $R^2$ and $R^4$ are methyl groups, and $R^3$ is OH.

**16.** A method for promoting hair growth, which comprises applying an effective amount of the hair growth promoting composition according to any of Claims 1 to 15 on skin of mammals.

**17.** The method for promoting hair growth according to claim 16, wherein the skin of mammals is human scalp.

# INTERNATIONAL SEARCH REPORT

International application No.
PCT/JP01/07538

A. CLASSIFICATION OF SUBJECT MATTER
Int.Cl$^7$ A61K7/06, A61P17/14

According to International Patent Classification (IPC) or to both national classification and IPC

B. FIELDS SEARCHED

Minimum documentation searched (classification system followed by classification symbols)
Int.Cl$^7$ A61K7/06, A61P17/14

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)
CAPLUS(STN)
REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | JP 11-263713 A (Shiseido Company, Limited. ),<br>28 September, 1999 (28.09.99),<br>Full text; especially, Claims 1,2 (Family: none) | 1,7,9,10,14,<br>16,17<br>1-17 |
| X<br>Y | WO 83/02390 A (HENKEL KOMMANDITGESELLSCHAFT AUF AKTIEN),<br>21 July, 1983 (21.07.83),<br>Full text; especially, Claims 1-3<br>& JP 59-500129 A | 1,2,14,16,17<br>1-17 |
| X<br>Y | JP 1-132510 A (Taisho Pharmaceutical Co., Ltd.),<br>25 May, 1989 (25.05.89),<br>Full text; especially, page 3, lower left column, experimentation example (Family: none) | 1,2,14,16,17<br>1-17 |
| X<br>Y | JP 1-132511 A (Lion Corporation),<br>25 May, 1989 (25.05.89),<br>Full text; especially, Claim 1; page 2, upper right column, formulas [I][II] (Family: none) | 1,14,16,17<br>1-17 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 December, 2001 (03.12.01) | 18 December, 2001 (18.12.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/07538

C (Continuation). DOCUMENTS CONSIDERED TO BE RELEVANT

| Category* | Citation of document, with indication, where appropriate, of the relevant passages | Relevant to claim No. |
|---|---|---|
| Y | JP 11-263711 A (Shiseido Company, Limited), 28 September, 1999 (28.09.99), Full text; especially, Claims 1-38; page 8, table 1 (Family: none) | 1-17 |
| A | JP 4-342415 A (Lion Corporation), 30 November, 1992 (30.11.92), Full text; especially, Claim 1 | 1,5,6,16,17 |

Form PCT/ISA/210 (continuation of second sheet) (July 1992)